# FASCICULE DE BREVET EUROPEEN

(11) **EP 3 106 134 B1**
(45) Date de publication et mention de la délivrance du brevet: **03.04.2019**
(21) Numéro de dépôt: 16174863.7
(22) Date de dépôt: 16.06.2016
(51) Int. Cl.: A61F 2/76, A61F 2/80

(54) **PIECE DE JONCTION ENTRE UNE EMBOÎTURE PROTHETIQUE ET UN MEMBRE PROTHETIQUE**
VERBINDUNGSTEIL ZWISCHEN EINEM PROTHESENSCHAFT UND EINEM PROTHESENGLIED
JOINING PART BETWEEN A PROSTHETIC SOCKET AND A PROSTHETIC LIMB

(30) Priorité: 18.06.2015 FR 1555578
(43) Date de publication de la demande: 21.12.2016
(73) Titulaire: Chabloz Composants, 38170 Seyssinet-Pariset (FR)
(72) Inventeur: Chabloz, Pierre, 38450 Saint Georges de Commiers (FR)
(74) Mandataire: Talbot, Alexandre

(56) Documents cités:
- US-A- 4 883 494
- US-A1- 2004 204 770
- US-A1- 2011 015 761
- US-A1- 2012 239 163
- US-A1- 2014 039 644

## Description

La présente invention a trait à une pièce de jonction entre une emboîture prothétique et un membre prothétique, à un ensemble prothétique comportant une telle pièce de jonction, ainsi qu'à une prothèse comportant un tel ensemble prothétique. Une telle pièce de jonction convient tout particulièrement pour une emboîture test ou provisoire.

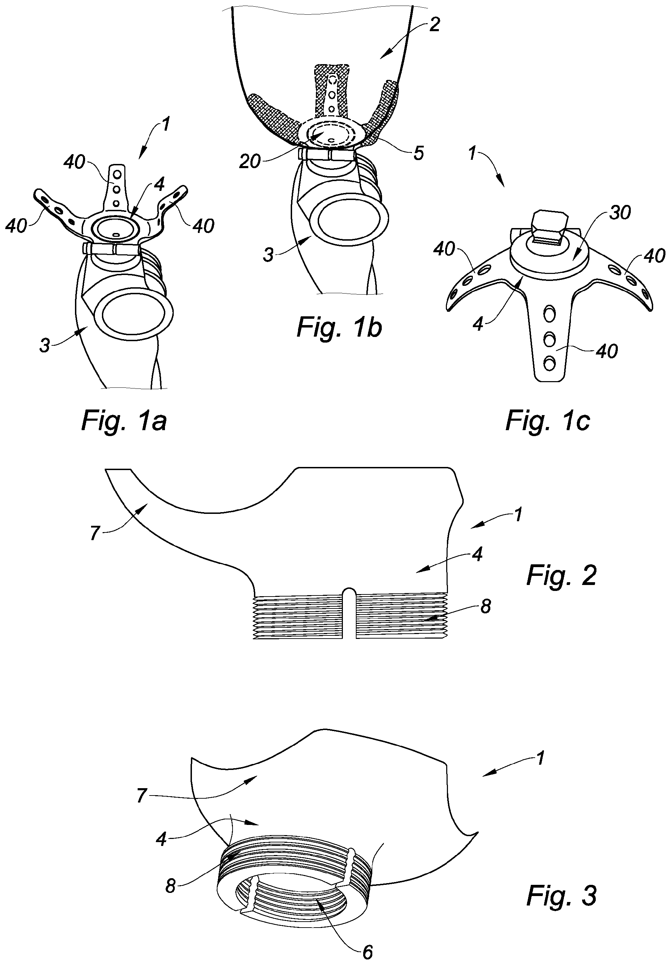

Une pièce de jonction connue de l'état de la technique, illustrée aux figure 1a à 1c, est une pièce de jonction 1 entre une emboîture prothétique 2 et un membre prothétique 3, l'emboîture prothétique 2 comportant une extrémité proximale ouverte destinée à recevoir un manchon et une extrémité distale 20 fermée, le membre prothétique 3 comportant un organe de fixation 30 muni d'un filetage, la pièce de jonction comportant :
- des moyens de réception adaptés pour recevoir l'extrémité distale 20 de l'emboîture prothétique 2, et comprenant une embase 4 ;
- un taraudage (non visible) ménagé dans l'embase 4, et adapté pour coopérer avec le filetage de l'organe de fixation 30 du membre prothétique 3.

Les moyens de réception comportent trois pattes de fixation 40 d'un matériau métallique, généralement un acier inoxydable, s'étendant à partir de l'embase 4. Les pattes de fixation 40 sont réparties autour de l'embase 4 selon un angle proche de 90°. Les pattes de fixation 40 sont déformables manuellement à l'aide de griffes. Les pattes de fixation 40 sont collées à l'emboîture prothétique 2 sur une table de transfert, et renforcées à l'aide de feuilles de carbone 5 comportant des fibres de carbone imprégnées d'une matrice thermodurcissable ou thermoplastique.

L'assemblage des pattes de fixation 40 à l'emboîture prothétique 2 est long, de l'ordre d'une heure, et inesthétique en raison de l'extrémité distale 20 de l'emboîture 2 qui demeure visible, de même que les feuilles de carbone 5. En outre, la pièce de jonction, en acier inoxydable, possède une masse assez importante.

On peut citer la demande de brevet américain US 2004/204770, qui divulgue un adaptateur prothétique, permettant une rotation et une translation d'un membre prothétique part rapport à un axe du membre prothétique, et la demande de brevet américain US 2011/015761, qui divulgue un adaptateur prothétique, comprenant une pièce de jonction munie d'un taraudage configuré pour recevoir un organe de fixation fileté prévu sur un membre prothétique. On peut également citer, la demande de brevet américain US 2012/0239163, qui divulgue une prothèse de jambe, comprenant un adaptateur compact muni d'une connexion ayant un taraudage destiné à recevoir une partie tibiale de la prothèse, et la demande de brevet américain US 2014/0039644, qui divulgue un système prothétique ajustable pour une jambe, comprenant des vis pour sécuriser un organe de fixation avec une pièce de jonction. On peut en outre citer le brevet américain US 4,883,494, qui divulgue une pièce de jonction pour une jambe prothétique, entre une emboîture et un membre prothétiques. Le membre prothétique comporte un organe de fixation, et la pièce comporte une coupelle et une embase à partir de laquelle une paroi supérieure de la coupelle s'étend. En outre, un orifice taraudé est ménagé dans l'embase, pour recevoir une vis de serrage afin de fixer une partie de la jambe prothétique. Mais ces pièces de jonction sont complexes.

Ainsi, la présente invention vise à remédier en tout ou partie aux inconvénients précités, et concerne à cet effet une pièce de jonction entre une emboîture prothétique et un membre prothétique telle que définie dans la revendication 1.

Ainsi, une pièce de jonction selon l'invention, grâce à une telle paroi supérieure, permet à la fois :
- de dissimuler l'extrémité distale de l'emboîture prothétique via l'enceinte formée par la paroi supérieure, et ce pour des raisons esthétiques ;
- d'augmenter la surface de contact de la paroi supérieure qui enveloppe l'extrémité distale de l'emboîture prothétique, et ce afin d'autoriser un collage plus simple et plus rapide que dans l'état de la technique.

Par « proximale », on entend l'extrémité qui est située la plus près du membre résiduel, le membre résiduel étant destiné à être enveloppé par le manchon.

Par « distale », on entend l'extrémité qui est la plus éloignée du membre résiduel.

Par « supérieure », on entend que la paroi s'étend au-dessus de l'embase en condition d'utilisation.

Selon une caractéristique générale de la présente invention, l'embase comporte un filetage s'étendant autour du taraudage, ledit filetage étant adapté pour coopérer avec un organe de blocage, de type écrou, pouvant bloquer en rotation de l'organe de fixation du membre prothétique.

Avantageusement, les moyens de réception sont d'un matériau plastique, le matériau plastique étant de préférence à base de polyamide.

Ainsi, le choix d'un matériau plastique permet d'alléger la pièce de jonction relativement à l'état de la technique et de réduire les coûts de fabrication. Le choix particulier du polyamide est particulièrement avantageux dans la mesure où il possède une durée de vie d'au moins 15 ans dans des conditions normales d'utilisation, et résiste à un environnement extérieur hostile (projection d'eau, immersion, corrosion, chaleur).

Avantageusement, la pièce de jonction est monobloc par moulage du matériau plastique.

Ainsi, une telle pièce de jonction est simple à réaliser, ne nécessitant pas d'inserts pour le filetage et le taraudage de l'embase.

Avantageusement, la paroi supérieure comporte une couche d'un mastic bi-composants agencée pour assembler la paroi supérieure à l'extrémité distale de l'emboîture prothétique.

Ainsi, une telle couche permet de coller rapidement, de l'ordre de 5 minutes, la pièce de jonction à l'emboîture, et de manière robuste grâce à la surface de contact importante de la paroi supérieure qui enveloppe l'extrémité distale de l'emboîture prothétique.

Selon une caractéristique, la paroi supérieure présente au moins une section transversale formant une courbe fermée.

Selon une caractéristique de l'invention, la paroi supérieure est agencée de sorte que l'enceinte est excentrée relativement à l'embase.

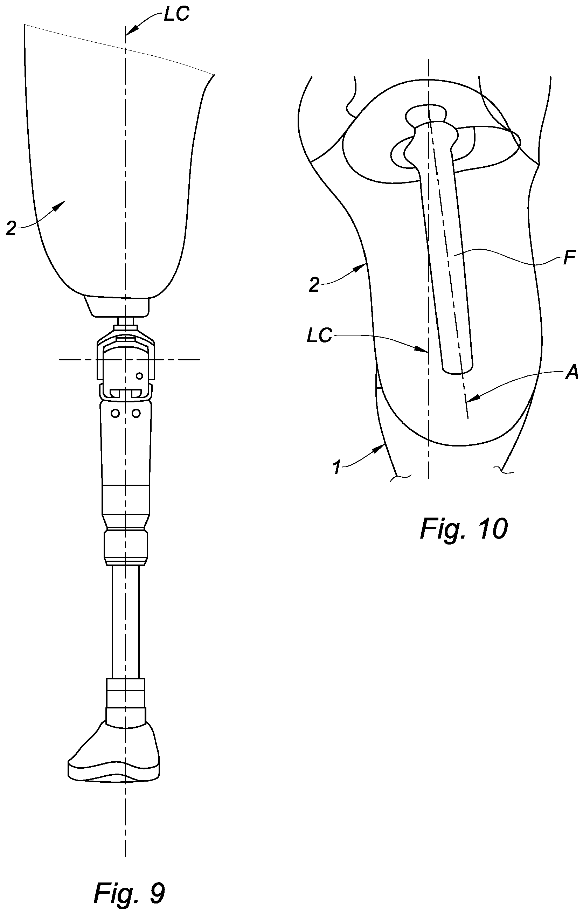

Dans le cas d'un genou prothétique, la ligne de charge doit généralement passer en avant du centre articulaire (ou de l'axe articulaire) du genou afin d'obtenir un genou prothétique verrouillé en extension. Comme illustré à la figure 9, en coupe frontale, la ligne de charge LC passe par le milieu de la hanche, le milieu du genou et le milieu de la cheville. La ligne de charge est donc destinée à passer par une zone centrale de l'embase. Or, comme illustré à la figure 10, le centre articulaire du genou n'est pas aligné avec l'axe A du moignon (correspondant également à l'axe longitudinal du fémur F). Ainsi, un avantage procuré par l'agencement excentré de l'enceinte relativement à l'embase est de faciliter l'assemblage de la pièce de jonction sur l'emboîture prothétique, en tenant compte du désalignement entre le centre articulaire du genou et l'axe du moignon. La ligne de charge peut donc passer aisément en avant du centre articulaire du genou.

En revanche, pour certains types de genoux prothétiques, la ligne de charge est proche de l'axe articulaire du genou. Or, les emboîtures prothétiques sont généralement alignées avec environ 5° de flexion. Il en résulte que la ligne de charge passe en arrière de l'axe du moignon. Dans ce cas, un avantage procuré par l'agencement excentré de l'enceinte relativement à l'embase est de faciliter l'assemblage de la pièce de jonction sur l'emboîture prothétique, en tenant compte du désalignement entre la ligne de charge et l'axe du moignon.

En outre, cet agencement excentré de l'enceinte relativement à l'embase est d'autant plus important que l'organe de fixation du membre prothétique (le terme « encre » est généralement employé) et la pièce de jonction ne sont pas montés sur l'emboîture prothétique de manière simultanée. En effet, l'encre est montée postérieurement sur l'emboîture prothétique.

Selon une caractéristique de l'invention, l'embase présente une section transversale possédant un premier centre de symétrie ; la paroi supérieure présente une section transversale possédant un deuxième centre de symétrie ; et les premier et deuxième centres de symétrie sont décalés d'une distance, suivant une direction transversale. Ladite distance est adaptée au désalignement entre le centre articulaire du genou et l'axe du moignon ou au désalignement entre la ligne de charge et l'axe du moignon le cas échéant.

Par « transversal(e) », on entend une direction perpendiculaire à la ligne de charge.

La présente invention concerne également un ensemble prothétique tel que défini dans la revendication 6, comportant :
- une emboîture prothétique comportant une extrémité proximale ouverte destinée à recevoir un manchon et une extrémité distale fermée ;
- une pièce de jonction conforme à l'invention, l'extrémité distale étant enveloppée par l'enceinte formée par la paroi supérieure.

Avantageusement, l'emboîture prothétique est à base de polytéréphtalate d'éthylène modifié glycol (PET-G) ou à base d'un polyépoxyde.

La présente invention concerne enfin une prothèse telle que définie dans la revendication 9, comportant :
- un ensemble prothétique conforme à l'invention,
- un membre prothétique, de préférence un genou prothétique, comportant un organe de fixation muni d'un filetage, l'organe de fixation étant agencé pour coopérer avec le taraudage de manière à fixer le membre prothétique à l'embase.

Avantageusement, la prothèse comporte un organe de blocage, de type écrou, agencé pour coopérer avec le filetage de l'embase de manière à bloquer en rotation l'organe de fixation du membre prothétique dans le taraudage de l'embase.

D'autres caractéristiques et avantages apparaîtront dans la description qui va suivre d'un mode de réalisation de l'invention donné à titre d'exemple non limitatif, ainsi que de différentes variantes de réalisation non couvertes par la présente invention, données à titre purement illustratif, en référence aux dessins annexés dans lesquels :
- les figures 1a à 1c (déjà commentées) sont des vues schématiques en perspective d'une pièce de jonction selon l'état de la technique,
- la figure 2 est une vue schématique de côté d'une pièce de jonction selon un mode de réalisation de l'invention,
- la figure 3 est une vue schématique en perspective, de dessous, d'une pièce de jonction selon ce mode de réalisation,
- la figure 4 est une vue schématique en perspective d'une pièce de jonction non couverte par la présente invention telle que définie par les revendications,
- la figure 5 est une vue schématique en coupe de la pièce de jonction illustrée à la figure 4,
- la figure 6 est une vue schématique de côté d'un écrou pouvant équiper une pièce de jonction selon l'invention,
- la figure 7 est une vue schématique en perspective d'une autre pièce de jonction non couverte par la présente invention telle que définie par les revendications,
- la figure 8 est une vue schématique en perspective de la pièce de jonction illustrée à la figure 7,
- la figure 9 est une vue schématique frontale d'un ensemble prothétique de l'état de la technique, illustrant la ligne de charge,
- la figure 10 est une vue schématique sagittale d'un moignon illustrant le désalignement entre le centre articulaire du genou et l'axe du moignon.

Dans la description qui suit, les mêmes références seront utilisées pour des éléments identiques ou assurant la même fonction, par souci de simplification de la description. Les caractéristiques techniques décrites ci-après pour différents exemples et mode de réalisation sont à considérer isolément ou selon toute combinaison techniquement possible.

La pièce de jonction illustrée aux figures 2 à 5 est une pièce de jonction 1 entre une emboîture prothétique (non illustrée) et un membre prothétique (non illustré), l'emboîture prothétique comportant une extrémité proximale ouverte destinée à recevoir un manchon et une extrémité distale fermée, le membre prothétique comportant un organe de fixation muni d'un filetage. L'emboîture prothétique est avantageusement à base de polytéréphtalate d'éthylène modifié glycol (PET-G) ou à base d'un polyépoxyde. Le membre prothétique est préférentiellement un genou prothétique. Le manchon est destiné à envelopper le membre résiduel.

La pièce de jonction 1 comporte :
- des moyens de réception adaptés pour recevoir l'extrémité distale de l'emboîture prothétique, et comprenant une embase 4 ;
- un taraudage 6 ménagé dans l'embase 4, et adapté pour coopérer avec le filetage de l'organe de fixation du membre prothétique.

Les moyens de réception comportent une paroi supérieure 7 s'étendant à partir de l'embase 4. La paroi supérieure 7 forme une enceinte adaptée pour envelopper l'extrémité distale de l'emboîture prothétique. L'enceinte formée par la paroi supérieure 7 comporte une surface interne 70 concave, de préférence fermée, et adaptée pour envelopper l'extrémité distale de l'emboîture prothétique. La paroi supérieure 7 présente avantageusement au moins une section transversale, par exemple sensiblement circulaire, formant une courbe fermée. La paroi supérieure 7 est avantageusement agencée de sorte que l'enceinte formée est excentrée relativement à l'embase 4.

La paroi supérieure 7 comporte avantageusement une couche d'un mastic bi-composants (non illustrée) agencée pour assembler la paroi supérieure 7 à l'extrémité distale de l'emboîture prothétique, le mastic bi-composants étant de préférence à base de polyuréthane.

Les moyens de réception sont avantageusement d'un matériau plastique, le matériau plastique étant de préférence à base de polyamide. En d'autres termes, l'embase 4 et la paroi supérieure 7 sont avantageusement du matériau plastique. La pièce de jonction 1 est avantageusement monobloc par moulage du matériau plastique, de préférence par injection. En d'autres termes, l'embase 4, la paroi supérieure 7, et le taraudage 6 sont avantageusement monobloc par moulage du matériau plastique.

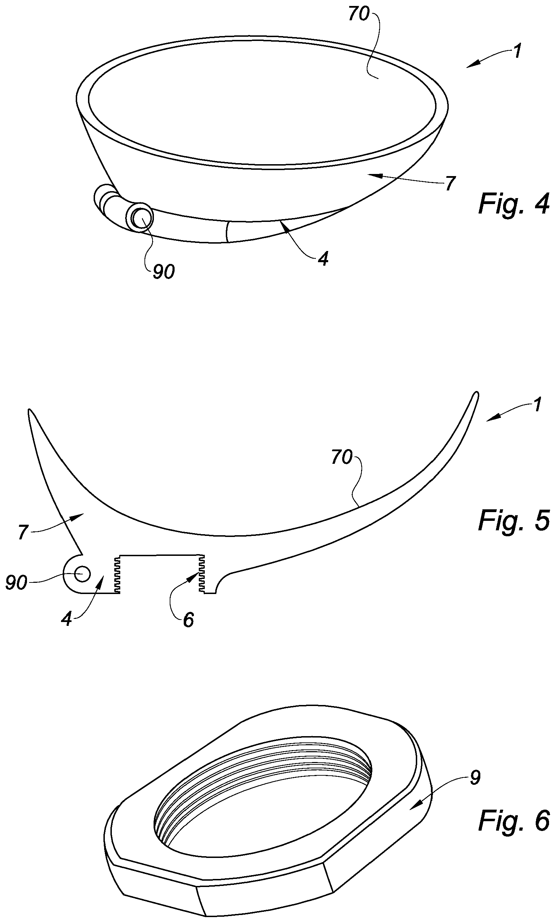

Selon la présente invention, l'embase 4 comporte avantageusement un filetage 8 s'étendant autour du taraudage 6, ledit filetage 8 étant adapté pour coopérer avec un organe de blocage 9 (illustré à la figure 6), de type écrou, pouvant bloquer en rotation de l'organe de fixation du membre prothétique. L'embase 4, la paroi supérieure 7, le taraudage 6 et le filetage 8 sont avantageusement monobloc par moulage du matériau plastique.

Dans une variante de réalisation illustrée aux figures 4 et 5, non couverte par la présente invention, le blocage en rotation de l'organe de fixation du membre prothétique est assuré par une vis 90, de préférence BTR, à six pans creux.

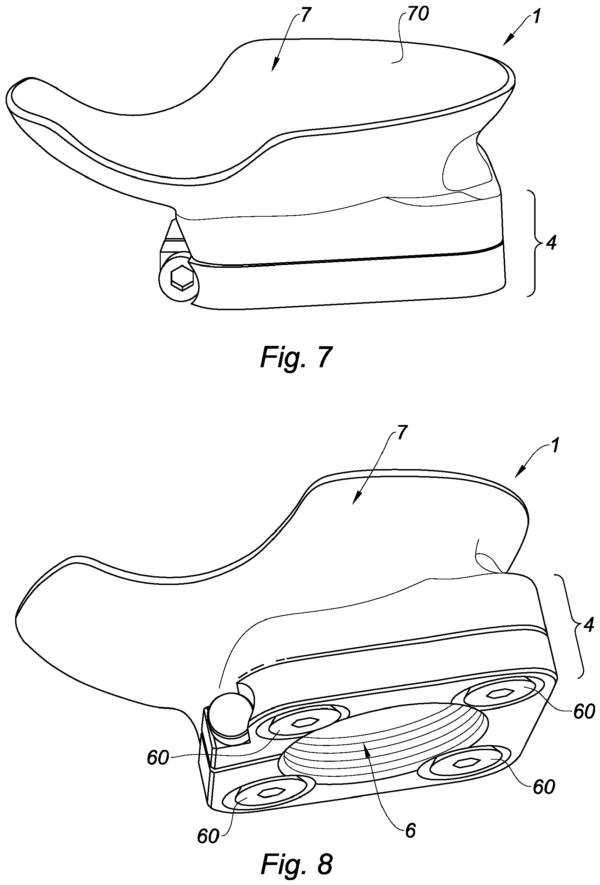

Dans une autre variante de réalisation illustrée aux figures 7 et 8, également non couverte par la présente invention, l'embase 4 de la pièce de jonction 1 comporte :
- une première partie, de préférence réalisée dans un matériau plastique ;
- une deuxième partie, de préférence de type insert métallique, muni d'un taraudage 6 central. Le taraudage 6 central est destiné à coopérer avec l'organe de fixation 30 de manière à fixer le membre prothétique 3 à la deuxième partie de l'embase 4.

La paroi supérieure 7 s'étend à partir de la première partie de l'embase 4. La deuxième partie de l'embase 4 comporte préférentiellement quatre taraudages périphériques ménagés autour du taraudage 6 central. La deuxième partie de l'embase 4 peut être rendue solidaire de la première partie de l'embase 4 par quatre vis de fixation 60 coopérant avec les quatre taraudages périphériques. Les taraudages périphériques présentent préférentiellement une section transversale oblongue, par exemple de forme ovale, de manière à autoriser des ajustements en translation de la deuxième partie de l'embase 4.

## Revendications

1. Pièce de jonction (1) entre une emboîture prothétique (2) et un membre prothétique (3), l'emboîture prothétique (2) comportant une extrémité proximale ouverte destinée à recevoir un manchon et une extrémité distale (20) fermée, le membre prothétique (3) comportant un organe de fixation (30) muni d'un filetage, la pièce de jonction (1) comportant :
- des moyens de réception adaptés pour recevoir l'extrémité distale (20) de l'emboîture prothétique (2), et comprenant une embase (4) ;
- un taraudage (6) ménagé dans l'embase (4), et adapté pour coopérer avec le filetage de l'organe de fixation (30) du membre prothétique (3) ;
- les moyens de réception comportant une paroi supérieure (7) s'étendant à partir de l'embase (4) et formant une enceinte adaptée pour envelopper l'extrémité distale (20) de l'emboîture prothétique (2), la paroi supérieure (7) étant agencée de sorte que l'enceinte est excentrée relativement à l'embase (4), **caractérisée en ce que** l'embase (4) comporte un filetage (8) s'étendant autour du taraudage (6), ledit filetage (8) étant adapté pour coopérer avec un organe de blocage (9) de type écrou pouvant bloquer en rotation l'organe de fixation (30) du membre prothétique (3).

2. Pièce de jonction (1) selon la revendication 1, **caractérisée en ce que** les moyens de réception sont d'un matériau plastique, le matériau plastique étant de préférence à base de polyamide.

3. Pièce de jonction (1) selon la revendication 2, **caractérisée en ce qu'**elle est monobloc par moulage du matériau plastique.

4. Pièce de jonction (1) selon l'une des revendications 1 à 3, **caractérisée en ce que** la paroi supérieure (7) comporte une couche d'un mastic bi-composants agencée pour assembler la paroi supérieure (7) à l'extrémité distale (20) de l'emboîture prothétique (2).

5. Pièce de jonction (1) selon l'une des revendications 1 à 4, **caractérisée en ce que** la paroi supérieure (7) présente au moins une section transversale formant une courbe fermée.

6. Ensemble prothétique comportant :
- une emboîture prothétique (2) comportant une extrémité proximale ouverte destinée à recevoir un manchon et une extrémité distale (20) fermée ;
- une pièce de jonction (1) selon l'une des revendications 1 à 5, l'extrémité distale (20) étant enveloppée par l'enceinte formée par la paroi supérieure (7).

7. Ensemble prothétique selon la revendication 6, **caractérisé en ce que** l'emboîture prothétique (2) est à base de polytéréphtalate d'éthylène modifié glycol (PET-G) ou à base d'un polyépoxyde.

8. Prothèse comportant :
- un ensemble prothétique (1, 2) selon la revendication 6 ou 7,
- un membre prothétique (3), de préférence un genou prothétique, comportant un organe de fixation (30) muni d'un filetage, l'organe de fixation (30) étant agencé pour coopérer avec le taraudage (6) de manière à fixer le membre prothétique (3) à l'embase (4).

9. Prothèse selon la revendication 8, **caractérisée en ce que** la prothèse comporte un organe de blocage (9), de préférence de type écrou, agencé pour coopérer avec le filetage (8) de l'embase (4) de manière à bloquer en rotation l'organe de fixation (30) du membre prothétique (3) dans le taraudage (6) de l'embase (4).

## Patentansprüche

1. Verbindungsteil (1) zwischen einem Prothesenschaft (2) und einem Prothesenglied (3), wobei der Prothesenschaft (2) ein proximales, offenes Ende umfasst, das dazu vorgesehen ist, eine Hülse aufzunehmen, sowie ein distales, geschlossenes Ende (20), wobei das Prothesenglied (3) eine mit einem Gewinde versehene Befestigungsvorrichtung (30) besitzt, welches Verbindungsteil (1) umfasst:
- Aufnahmevorrichtungen, die geeignet sind, das distale Ende (20) des Prothesenschafts (2) aufzunehmen, und einen Sockel (4) umfassen;
- in dem Sockel (4) ein Gewinde (6), das geeignet ist, mit dem Gewinde der Befestigungsvorrichtung (30) des Prothesenglieds (3) zusammenzuwirken;
- die Aufnahmemittel eine obere Wand (7) umfassen, die vom Sockel (4) ausgeht und ein Gehäuse bildet, das geeignet ist, das distale Ende (20) des Prothesenschafts (2) zu umgeben, wobei die obere Wand (7) so vorgesehen ist, dass das Gehäuse bezüglich dem Sockel (4) exzentrisch ist, **dadurch gekennzeichnet, dass** der Sockel (4) ein Gewinde (8) umfasst, das sich um das Gewinde (6) herum erstreckt, wobei das Gewinde (8) geeignet ist, mit einer Arretierungsvorrichtung (9) nach Art einer Mutter zusammenzuwirken, um die Befestigungsvorrichtung (30) des Prothesenglieds (3) in seiner Drehung zu arretieren.

2. Verbindungsteil (1) nach Anspruch 1, **dadurch gekennzeichnet, dass** die Aufnahmemittel aus einem Kunststoff bestehen, wobei der Kunststoff vorzugsweise auf Polyamid basiert.

3. Verbindungsteil (1) nach Anspruch 2, **dadurch gekennzeichnet, dass** es durch Formguss des Kunststoffs aus einem Stück besteht.

4. Verbindungsteil (1) nach einem der Ansprüche 1 bis 3, **dadurch gekennzeichnet, dass** die obere Wand (7) eine Schicht aus einem Bikomponenten-Kitt umfasst, die so vorgesehen ist, dass sie die obere Wand (7) mit dem distalen Ende (20) des Prothesenschafts (2) verbindet.

5. Verbindungsteil (1) nach einem der Ansprüche 1 bis 4, **dadurch gekennzeichnet, dass** die obere Wand (7) mindestens einen Querschnitt aufweist, der eine geschlossene Kurve bildet.

6. Protheseneinheit, die umfasst:
- einen Prothesenschaft (2), der ein proximales, offenes Ende umfasst, das vorgesehen ist, eine Hülse und ein distales, geschlossenes Ende (20) aufzunehmen;
- ein Verbindungsteil (1) nach einem der Ansprüche 1 bis 5, wobei das distale Ende (20) von dem von der oberen Wand (7) gebildeten Gehäuse umgeben ist.

7. Protheseneinheit nach Anspruch 6, **dadurch gekennzeichnet, dass** der Prothesenschaft (2) auf Basis eines Glykol-modifiziertem Polyethylenterephthalats (PET-G) oder eines Polyepoxids hergestellt ist.

8. Prothese, die umfasst:
- eine Protheseneinheit (2) nach Anspruch 6 oder 7,
- ein Prothesenglied (3), vorzugsweise eine Knieprothese, umfassend ein Befestigungselement (30), das mit einem Gewinde versehen ist, wobei die Befestigungsvorrichtung (30) so vorgesehen ist, dass sie mit dem Gewinde (6) so zusammenwirkt, dass sie das Prothesenglied (3) am Sockel (4) befestigt.

9. Prothese nach Anspruch 8, **dadurch gekennzeichnet, dass** die Prothese ein Arretierungselement (9) aufweist, vorzugsweise ein Element nach Art einer Mutter, das so vorgesehen ist, dass es mit dem Gewinde (8) des Sockels (4) zusammenwirkt, um die Befestigungsvorrichtung (30) des Prothesenglieds (3) in dem Gewinde (6) des Sockels in Drehung zu arretieren.

## Claims

1. Joining part (1) between a prosthetic socket (2) and a prosthetic limb (3), the prosthetic socket (2) comprising an open proximal end designed to receive a sleeve and a closed distal end (20), the prosthetic limb (3) comprising a fixing means (30) provided with a thread, the joining part (1) comprising:
- receiving means designed to receive the distal end (20) of the prosthetic socket (2), and comprising a base (4);
- a tapping (6) provided in the base (4), and designed to operate in collaboration with the thread of the fixing means (30) of the prosthetic limb (3);
- the receiving means comprising an upper wall (7) extending from the base (4) and forming a suitable enclosure for enclosing the distal end (20) of the prosthetic socket (2), the upper wall (7) being arranged so that the enclosure is eccentric relatively to the base (4),
**characterised in that** the base (4) comprises a thread (8) extending around the tapping (6), said thread (8) being designed to operate in collaboration with a securing means (9), of nut type designed to prevent movement of the fixing means (30) of the prosthetic limb (3) in rotation.

2. Joining part (1) according to claim 1, **characterised in that** the receiving means are made from a plastic material, the plastic material preferably being made from a polyamide base.

3. Joining part (1) according to claim 2, **characterised in that** it is a monobloc part made by moulding of the plastic material.

4. Joining part (1) according to one of claims 1 to 3, **characterised in that** the upper wall (7) comprises a layer of a two-component sealant arranged to assemble the upper wall (7) to the distal end (20) of the prosthetic socket (2).

5. Joining part (1) according to one of claims 1 to 4, **characterised in that** the upper wall (7) presents at least a transverse cross-section forming a closed curve.

6. Prosthetic assembly comprising:
- a prosthetic socket (2) comprising an open proximal end designed to receive a sleeve and a closed distal end (20);
- a joining part (1) according to one of claims 1 to 5, the distal end (20) being enclosed by the enclosure formed by the upper wall (7).

7. Prosthetic assembly according to claim 6, **characterised in that** the prosthetic socket (2) is made from polyethylene terephtalate glycol-modified (PET-G) or from a polyepoxide.

8. Prosthesis comprising:
- a prosthetic assembly (1, 2) according to claim 6 or 7,
- a prosthetic limb (3), preferably a prosthetic knee, comprising a fixing means (30) provided with a thread, the fixing means (30) being arranged to operate in collaboration with the tapping (6) so as to secure the prosthetic limb (3) to the base (4).

9. Prosthesis according to claim 8, **characterised in that** the prosthesis comprises a securing means (9), preferably of nut type, arranged to operate in collaboration with the thread (8) of the base (4) so as to prevent movement of the fixing means (30) of the prosthetic limb (3) in rotation in the tapping (6) of the base (4).
